# EUROPEAN PATENT APPLICATION

(11) **EP 0 725 078 A1**
(43) Date of publication of application: **07.08.1996**
(21) Application number: 96300612.7
(22) Date of filing: 29.01.1996
(51) Int. Cl.: C07K 14/47, A61K 38/17

(54) **Anti-obesity proteins**

(30) Priority: 31.01.1995 US 381048; 06.02.1995 US 383638
(71) Applicant: ELI LILLY AND COMPANY, Indianapolis, Indiana 46285 (US)
(72) Inventor: Basinsky, Margret B., Indianapolis, Indiana 46219 (US); Dimarchi, Richard D., Carmel, Indiana 46033 (US); Heath Jr., William F., Fishers, Indiana 46038 (US); Schoner, Brigitte E., Monrovia, Indiana 46157 (US)
(74) Representative: Tapping, Kenneth George

(57) **Abstract**

The present invention provides anti-obesity proteins, which when administered to a patient regulate fat tissue. Peptides of ivention are represented by mentioned DNA string or analogs thereof: 
5                   10                  15
Val Pro Ile Gln Lys Val Gln Asp Asp Thr Lys Thr Leu Ile Lys Thr

            20                  25                  30
Ile Val Thr Arg Ile Asp Asp Ile Ser His Thr Gln Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Val Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Trp Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys
 Accordingly, such agents allow patients to overcome their obesity handicap and live normal lives with much reduced risk for type II diabetes, cardiovascular disease and cancer.

## Description

The present invention is in the field of human medicine, particularly in the treatment of obesity and disorders associated with obesity. Most specifically the invention relates to anti-obesity proteins that when administered to a patient regulate fat tissue.

Obesity, and especially upper body obesity, is a common and very serious public health problem in the United States and throughout the world. According to recent statistics, more than 25% of the United States population and 27% of the Canadian population are over weight. Kuczmarski, Amer. J. of Clin. Nut. 55: 495S - 502S (1992); Reeder et. al., Can. Med. Ass. J., 23: 226-233 (1992). Upper body obesity is the strongest risk factor known for type II diabetes mellitus, and is a strong risk factor for cardiovascular disease and cancer as well. Recent estimates for the medical cost of obesity are $150,000,000,000 world wide. The problem has become serious enough that the surgeon general has begun an initiative to combat the ever increasing adiposity rampant in American society.

Much of this obesity induced pathology can be attributed to the strong association with dyslipidemia, hypertension, and insulin resistance. Many studies have demonstrated that reduction in obesity by diet and exercise reduces these risk factors dramatically. Unfortunately these treatments are largely unsuccessful with a failure rate reaching 95%. This failure may be due to the fact that the condition is strongly associated with genetically inherited factors that contribute to increased appetite, preference for highly caloric foods, reduced physical activity, and increased lipogenic metabolism. This indicates that people inheriting these genetic traits are prone to becoming obese regardless of their efforts to combat the condition. Therefore, a new pharmacological agent that can correct this adiposity handicap and allow the physician to successfully treat obese patients in spite of their genetic inheritance is needed.

The *ob / ob* mouse is a model of obesity and diabetes that is known to carry an autosomal recessive trait linked to a mutation in the sixth chromosome. Recently, Yiying Zhang and co-workers published the positional cloning of the mouse gene linked with this condition. Yiying Zhang et al. Nature 372: 425-32 (1994). This report disclosed a gene coding for a 167 amino acid protein with a 21 amino acid signal peptide that is exclusively expressed in adipose tissue.

Physiologist have postulated for years that, when a mammal overeats, the resulting excess fat signals to the brain that the body is obese which, in turn, causes the body to eat less and burn more fuel. G. R. Hervey, Nature 227: 629-631 (1969). This "feedback" model is supported by parabiotic experiments, which implicate a circulating hormone controlling adiposity. Based on this model, the protein, which is apparently encoded by the *ob* gene, is now speculated to be an adiposity regulating hormone.

Pharmacological agents which are biologically active and mimic the activity of this protein are useful to help patients regulate their appetite and metabolism and thereby control their adiposity. Until the present invention, such a pharmacological agent was unknown.

The present invention provides biologically active anti-obesity proteins. Such agents therefore allow patients to overcome their obesity handicap and live normal lives with a more normalized risk for type II diabetes, cardiovascular disease and cancer.

### Summary of Invention

The present invention is directed to a biologically active anti-obesity protein of the Formula (I): 
(SEQ ID NO: 1)
5 10 15
Val Pro Ile Xaa Lys Val Xaa Asp Asp Thr Lys Thr Leu Ile Lys Thr
20 25 30
Ile Val Thr Arg Ile Xaa Asp Ile Ser His Xaa Xaa Ser Val Ser Ser
 
35 40 45
Lys Xaa Lys Val Thr Gly Leu Asp Phe Ile Pro Gly Leu His Pro Ile

50 55 60
Leu Thr Leu Ser Lys Xaa Asp Xaa Thr Leu Ala Val Tyr Xaa Xaa Ile

65 70 75 80
Leu Thr Ser Xaa Pro Ser Arg Xaa Val Ile Xaa Ile Ser Xaa Asp Leu

85 90 95
Glu Xaa Leu Arg Asp Leu Leu His Val Leu Ala Phe Ser Lys Ser Cys

100 105 110
His Leu Pro Xaa Ala Ser Gly Leu Glu Thr Leu Xaa Ser Leu Gly Gly

115 120 125
Val Leu Glu Ala Ser Gly Tyr Ser Thr Glu Val Val Ala Leu Ser Arg

130 135 140
Leu Xaa Gly Ser Leu Xaa Asp Xaa Leu Xaa Xaa Leu Asp Leu Ser Pro

145
Gly Cys

(I)
 wherein:
Xaa at position 4 is Gln or Glu;
Xaa at position 7 is Gln or Glu;
Xaa at position 22 is Gln, Asn, or Asp;
Xaa at position 27 is Thr or Ala;
Xaa at position 28 is Gln, Glu, or absent;
Xaa at position 34 is Gln or Glu;
Xaa at position 54 is Met, methionine sulfoxide, Leu, Ile, Val, Ala, or Gly;
Xaa at position 56 is Gln or Glu;
Xaa at position 62 is Gln or Glu;
Xaa at position 63 is Gln or Glu;
Xaa at position 68 is Met, methionine sulfoxide, Leu, Ile, Val, Ala, or Gly;
Xaa at position 72 is Gln, Asn, or Asp;
Xaa at position 75 is Gln or Glu;
Xaa at position 78 is Gln, Asn, or Asp;
Xaa at position 82 is Gln, Asn, or Asp;
Xaa at position 100 is Glu, Trp, Phe, Ile, Val, or Leu;
Xaa at position 108 is Asp or Glu;
Xaa at position 130 is Gln or Glu;
Xaa at position 134 is Gln or Glu;
Xaa at position 136 is Met, methionine sulfoxide, Leu, Ile, Val, Ala, or Gly;
Xaa at position 138 is Gln, Trp, Tyr, Phe, Ile, Val, or Leu;
Xaa at position 139 is Gln or Glu;
with the exception of compounds wherein:
Xaa at position 4 is Gln;
Xaa at position 7 is Gln;
Xaa at position 22 is Asn;
Xaa at position 27 is Thr;
Xaa at position 28 is Gln or absent;
Xaa at position 34 is Gln;
Xaa at position 54 is Met;
Xaa at position 56 is Gln;
Xaa at position 62 is Gln;
Xaa at position 63 is Gln;
Xaa at position 68 is Met;
Xaa at position 72 is Asn;
Xaa at position 75 is Gln;
Xaa at position 78 is Asn;
Xaa at position 82 is Asn;
Xaa at position 100 is Trp;
Xaa at position 108 is Asp;
Xaa at position 130 is Gln;
Xaa at position 134 is Gln;
Xaa at position 136 is Met;
Xaa at position 138 is Trp; and
Xaa at position 139 is Gln.

The invention further provides a method of treating obesity, which comprises administering to a mammal in need thereof a protein of the Formula (I).

The invention further provides a pharmaceutical formulation, which comprises a protein of the Formula (I) together with one or more pharmaceutical acceptable diluents, carriers or excipients therefor.

### Detailed Description

For purposes of the present invention, as disclosed and claimed herein, the following terms and abbreviations are defined as follows:

Base pair (bp) -- refers to DNA or RNA. The abbreviations A,C,G, and T correspond to the 5'-monophosphate forms of the nucleotides (deoxy)adenine, (deoxy)cytidine, (deoxy)guanine, and (deoxy)thymine, respectively, when they occur in DNA molecules. The abbreviations U,C,G, and T correspond to the 5'-monophosphate forms of the nucleosides uracil, cytidine, guanine, and thymine, respectively when they occur in RNA molecules. In double stranded DNA, base pair may refer to a partnership of A with T or C with G. In a DNA/RNA heteroduplex, base pair may refer to a partnership of T or U with A or C with G.

Chelating Peptide -- An amino acid sequence capable of complexing with a multivalent metal ion.

Immunoreactive Protein(s) -- a term used to collectively describe antibodies, fragments of antibodies capable of binding antigens of a similar nature as the parent antibody molecule from which they are derived, and single chain polypeptide binding molecules as described in PCT Application No. PCT/US 87/02208, International Publication No. WO 88/01649.

MWCO -- an abbreviation for molecular weight cutoff.

Plasmid -- an extrachromosomal self-replicating genetic element.

PMSF -- an abbreviation for phenylmethylsulfonyl fluoride.

Reading frame -- the nucleotide sequence from which translation occurs "read" in triplets by the translational apparatus of tRNA, ribosomes and associated factors, each triplet corresponding to a particular amino acid. Because each triplet is distinct and of the same length, the coding sequence must be a multiple of three. A base pair insertion or deletion (termed a frameshift mutation) may result in two different proteins being coded for by the same DNA segment. To insure against this, the triplet codons corresponding to the desired polypeptide must be aligned in multiples of three from the initiation codon, i.e. the correct "reading frame" must be maintained. In the creation of fusion proteins containing a chelating peptide, the reading frame of the DNA sequence encoding the structural protein must be maintained in the DNA sequence encoding the chelating peptide.

Recombinant DNA Cloning Vector -- any autonomously replicating agent including, but not limited to, plasmids and phages, comprising a DNA molecule to which one or more additional DNA segments can or have been added.

Recombinant DNA Expression Vector -- any recombinant DNA cloning vector in which a promoter has been incorporated.

Replicon -- A DNA sequence that controls and allows for autonomous replication of a plasmid or other vector.

Transcription -- the process whereby information contained in a nucleotide sequence of DNA is transferred to a complementary RNA sequence.

Translation -- the process whereby the genetic information of messenger RNA is used to specify and direct the synthesis of a polypeptide chain.

Tris -- an abbreviation for tris(hydroxymethyl)aminomethane.

Treating -- describes the management and care of a patient for the purpose of combating the disease, condition, or disorder and includes the administration of a compound of present invention to prevent the onset of the symptoms or complications, alleviating the symptoms or complications, or eliminating the disease, condition, or disorder. Treating obesity therefor includes the inhibition of food intake, the inhibition of weight gain, and inducing weight loss in patients in need thereof.

Vector -- a replicon used for the transformation of cells in gene manipulation bearing polynucleotide sequences corresponding to appropriate protein molecules which, when combined with appropriate control sequences, confer specific properties on the host cell to be transformed. Plasmids, viruses, and bacteriophage are suitable vectors, since they are replicons in their own right. Artificial vectors are constructed by cutting and joining DNA molecules from different sources using restriction enzymes and ligases. Vectors include Recombinant DNA cloning vectors and Recombinant DNA expression vectors.

X-gal -- an abbreviation for 5-bromo-4-chloro-3-indolyl beta-D-galactoside.

As noted above, the present invention provides a protein of the Formula (I). The preferred proteins of the present invention are those of Formula (I) wherein Xaa at position 27 is Ala. Other preferred proteins of the present invention are those wherein: Xaa at position 108 is Asp; Xaa at position 22 is Asp; Xaa at position 100 is Trp or Gln; or Xaa at position 138 is Trp.

Other preferred proteins of the present invention are those of Formula (I) wherein:
Xaa at position 4 is Gln or Glu;
Xaa at position 7 is Gln or Glu;
Xaa at position 22 is Gln, Asn, or Asp;
Xaa at position 27 is Thr or Ala;
Xaa at position 28 is Gln or Glu;
Xaa at position 34 is Gln or Glu;
Xaa at position 54 is Met or methionine sulfoxide;
Xaa at position 56 is Gln or Glu;
Xaa at position 62 is Gln or Glu;
Xaa at position 63 is Gln or Glu;
Xaa at position 68 is Met or methionine sulfoxide;
Xaa at position 72 is Gln, Asn, or Asp;
Xaa at position 75 is Gln or Glu;
Xaa at position 78 is Gln, Asn, or Asp;
Xaa at position 100 is Trp;
Xaa at position 82 is Gln, Asn, or Asp;
Xaa at position 108 is Asp or Glu;
Xaa at position 130 is Gln or Glu;
Xaa at position 134 is Gln or Glu;
Xaa at position 136 is Met or methionine sulfoxide; and
Xaa at position 138 is Trp;
Xaa at position 139 is Gln or Glu.

The most preferred proteins of the present invention are those of Formula (I) wherein:
Xaa at position 4 is Gln;
Xaa at position 7 is Gln;
Xaa at position 22 is Gln, Asn, or Asp;
Xaa at position 27 is Thr or Ala;
Xaa at position 28 is Gln;
Xaa at position 34 is Gln;
Xaa at position 54 is Met;
Xaa at position 56 is Gln;
Xaa at position 62 is Gln;
Xaa at position 63 is Gln;
Xaa at position 68 is Met;
Xaa at position 72 is Gln, Asn, or Asp;
Xaa at position 75 is Gln;
Xaa at position 78 is Asn;
Xaa at position 82 is Gln, Asn, or Asp;
Xaa at position 100 is Trp;
Xaa at position 108 is Asp;
Xaa at position 130 is Gln;
Xaa at position 134 is Gln;
Xaa at position 136 is Met; and
Xaa at position 138 is Trp;
Xaa at position 139 is Gln.

Yet additional preferred proteins include those of Formula (I) wherein:
Xaa at position 4 is Gln or Glu;
Xaa at position 7 is Gln or Glu;
Xaa at position 22 is Asn;
Xaa at position 27 is Thr or Ala;
Xaa at position 28 is Gln or Glu;
Xaa at position 34 is Gln or Glu;
Xaa at position 54 is Met;
Xaa at position 56 is Gln or Glu;
Xaa at position 62 is Gln or Glu;
Xaa at position 63 is Gln or Glu;
Xaa at position 68 is Met;
Xaa at position 72 is Asn;
Xaa at position 75 is Gln or Glu;
Xaa at position 78 is Asn;
Xaa at position 82 is Asn;
Xaa at position 100 is Trp;
Xaa at position 108 is Asp;
Xaa at position 130 is Gln or Glu;
Xaa at position 134 is Gln or Glu;
Xaa at position 136 is Met; and
Xaa at position 138 is Trp;
Xaa at position 139 is Gln or Glu.
And those of Formula (I) wherein:
Xaa at position 4 is Gln;
Xaa at position 7 is Gln;
Xaa at position 22 is Asn;
Xaa at position 27 is Thr or Ala;
Xaa at position 28 is Gln;
Xaa at position 34 is Gln;
Xaa at position 54 is Met or methionine sulfoxide;
Xaa at position 56 is Gln;
Xaa at position 62 is Gln;
Xaa at position 63 is Gln;
Xaa at position 68 is Met or methionine sulfoxide;
Xaa at position 72 is Asn;
Xaa at position 75 is Gln;
Xaa at position 78 is Asn;
Xaa at position 82 is Asn;
Xaa at position 100 is Trp;
Xaa at position 108 is Asp;
Xaa at position 130 is Gln;
Xaa at position 134 is Gln;
Xaa at position 136 is Met or methionine sulfoxide; and
Xaa at position 138 is Trp;
Xaa at position 139 is Gln.

Preferred species of the present invention include those of the SEQ ID NO: 2 through 5.

(SEQ ID NO: 2)
5 10 15
Val Pro Ile Gln Lys Val Gln Asp Asp Thr Lys Thr Leu Ile Lys Thr

20 25 30
Ile Val Thr Arg Ile Asp Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Trp Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys

(SEQ ID NO: 3)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65                  70                  75                  80
Leu Thr Ser Met Pro Ser Arg Asn Val Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Gln Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys

(SEQ ID NO: 4)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Trp Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Gln Gln Leu Asp Leu Ser Pro

145
Gly Cys

(SEQ ID NO: 5)
5 10 15
Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Gln Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Gln Gln Leu Asp Leu Ser Pro

145
Gly Cys

The amino acids abbreviations are accepted by the United States Patent and Trademark Office as set forth in 37 C.F.R. § 1.822 (b) (2) (1993). One skilled in the art would recognize that certain amino acids are prone to rearrangement. For example, Asp may rearrange to aspartimide and isoasparigine as described in I. Schön et al., Int. J. Peptide Protein Res. 14: 485-94 (1979) and references cited therein. These rearrangement derivatives are included within the scope of the present invention. Unless otherwise indicated the amino acids are in the L configuration.

Yiying Zhang et al. in Nature 372: 425-32 (December 1994) report the cloning of the murine obese (*ob*) mouse gene and present mouse DNA and the naturally occurring amino acid sequence of the obesity protein for the mouse and human. This protein is speculated to be a hormone that is secreted by fat cells and controls body weight. No pharmacological activity is demonstrated by Zhang et al.

The present invention provides biologically active proteins that provide effective treatment for obesity. Many of the claimed proteins offer additional advantages of stability, especially acid stability, and improved absorption characteristics.

The claimed proteins ordinarily are prepared by modification of the DNA encoding the claimed protein and thereafter expressing the DNA in recombinant cell culture. Techniques for making substitutional mutations at predetermined sites in DNA having a known sequence are well known, for example M13 primer mutagenesis. The mutations that might be made in the DNA encoding the present anti-obesity proteins must not place the sequence out of reading frame and preferably will not create complementary regions that could produce secondary mRNA structure. See DeBoer et al., EP 75,444A (1983).

The compounds of the present invention may be produced either by recombinant DNA technology or well known chemical procedures, such as solution or solid-phase peptide synthesis, or semi-synthesis in solution beginning with protein fragments coupled through conventional solution methods.

### A. Solid Phase

The synthesis of the claimed protein may proceed by solid phase peptide synthesis or by recombinant methods. The principles of solid phase chemical synthesis of polypeptides are well known in the art and may be found in general texts in the area such as Dugas, H. and Penney, C., Bioorganic Chemistry Springer-Verlag, New York, pgs. 54-92 (1981). For example, peptides may be synthesized by solid-phase methodology utilizing an PE-Applied Biosystems 430A peptide synthesizer (commercially available from Applied Biosystems, Foster City California) and synthesis cycles supplied by Applied Biosystems. Boc amino acids and other reagents are commercially available from PE-Applied Biosystems and other chemical supply houses. Sequential Boc chemistry using double couple protocols are applied to the starting p-methyl benzhydryl amine resins for the production of C-terminal carboxamides. For the production of C-terminal acids, the corresponding PAM resin is used. Arginine, Asparagine, Glutamine, Histidine and Methionine are coupled using preformed hydroxy benzotriazole esters. The following side chain protection may be used:
Arg, Tosyl
Asp, cyclohexyl or benzyl
Cys, 4-methylbenzyl
Glu, cyclohexyl
His, benzyloxymethyl
Lys, 2-chlorobenzyloxycarbonyl
Met, sulfoxide
Ser, Benzyl
Thr, Benzyl
Trp, formyl
Tyr, 4-bromo carbobenzoxy
Boc deprotection may be accomplished with trifluoroacetic acid (TFA) in methylene chloride. Formyl removal from Trp is accomplished by treatment of the peptidyl resin with 20% piperidine in dimethylformamide for 60 minutes at 4°C. Met(O) can be reduced by treatment of the peptidyl resin with TFA/dimethylsulfide/conHCl (95/5/1) at 25°C for 60 minutes. Following the above pre-treatments, the peptides may be further deprotected and cleaved from the resin with anhydrous hydrogen fluoride containing a mixture of 10% m-cresol or m-cresol/10% p-thiocresol or m-cresol/p-thiocresol/dimethylsulfide. Cleavage of the side chain protecting group(s) and of the peptide from the resin is carried out at zero degrees Centigrade or below, preferably -20°C for thirty minutes followed by thirty minutes at 0°C. After removal of the HF, the peptide/resin is washed with ether. The peptide is extracted with glacial acetic acid and lyophilized. Purification is accomplished by reverse-phase C18 chromatography (Vydac) column in .1% TFA with a gradient of increasing acetonitrile concentration.

One skilled in the art recognizes that the solid phase synthesis could also be accomplished using the FMOC strategy and a TFA/scavenger cleavage mixture.

### B. Recombinant Synthesis

The claimed proteins may also be produced by recombinant methods. Recombinant methods are preferred if a high yield is desired. The basic steps in the recombinant production of protein include:
a) construction of a synthetic or semi-synthetic (or isolation from natural sources) DNA encoding the claimed protein,
b) integrating the coding sequence into an expression vector in a manner suitable for the expression of the protein either alone or as a fusion protein,
c) transforming an appropriate eukaryotic or prokaryotic host cell with the expression vector, and
d) recovering and purifying the recombinantly produced protein.

### 2.a. Gene Construction

Synthetic genes, the in vitro or in vivo transcription and translation of which will result in the production of the protein may be constructed by techniques well known in the art. Owing to the natural degeneracy of the genetic code, the skilled artisan will recognize that a sizable yet definite number of DNA sequences may be constructed which encode the claimed proteins. In the preferred practice of the invention, synthesis is achieved by recombinant DNA technology.

Methodology of synthetic gene construction is well known in the art. For example, see Brown, et al. (1979) Methods in Enzymology, Academic Press, N.Y., Vol. 68, pgs. 109-151. The DNA sequence corresponding to the synthetic claimed protein gene may be generated using conventional DNA synthesizing apparatus such as the Applied Biosystems Model 380A or 380B DNA synthesizers (commercially available from Applied Biosystems, Inc., 850 Lincoln Center Drive, Foster City, CA 94404).

It may desirable in some applications to modify the coding sequence of the claimed protein so as to incorporate a convenient protease sensitive cleavage site, e.g., between the signal peptide and the structural protein facilitating the controlled excision of the signal peptide from the fusion protein construct.

The gene encoding the claimed protein may also be created by using polymerase chain reaction (PCR). The template can be a cDNA library (commercially available from CLONETECH or STRATAGENE) or mRNA isolated from human adipose tissue. Such methodologies are well known in the art Maniatis, et al. Molecular Cloning: A Laboratory Manual, Cold Spring Harbor Press, Cold Spring Harbor Laboratory, Cold Spring Harbor, New York (1989).

### 2.b. Direct expression or Fusion protein

The claimed protein may be made either by direct expression or as fusion protein comprising the claimed protein followed by enzymatic or chemical cleavage. A variety of peptidases (e.g. trypsin) which cleave a polypeptide at specific sites or digest the peptides from the amino or carboxy termini (e.g. diaminopeptidase) of the peptide chain are known. Furthermore, particular chemicals (e.g. cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. See e.g., Carter P., Site Specific Proteolysis of Fusion Proteins, Ch. 13 in Protein Purification: From Molecular Mechanisms to Large Scale Processes, American Chemical Soc., Washington, D.C. (1990).

### 2.c. Vector Construction

Construction of suitable vectors containing the desired coding and control sequences employ standard ligation techniques. Isolated plasmids or DNA fragments are cleaved, tailored, and religated in the form desired to form the plasmids required.

To effect the translation of the desired protein, one inserts the engineered synthetic DNA sequence in any of a plethora of appropriate recombinant DNA expression vectors through the use of appropriate restriction endonucleases. The claimed protein is a relatively large protein. A synthetic coding sequence is designed to possess restriction endonuclease cleavage sites at either end of the transcript to facilitate isolation from and integration into these expression and amplification and expression plasmids. The isolated cDNA coding sequence may be readily modified by the use of synthetic linkers to facilitate the incorporation of this sequence into the desired cloning vectors by techniques well known in the art. The particular endonucleases employed will be dictated by the restriction endonuclease cleavage pattern of the parent expression vector to be employed. The choice of restriction sites are chosen so as to properly orient the coding sequence with control sequences to achieve proper in-frame reading and expression of the claimed protein.

In general, plasmid vectors containing promoters and control sequences which are derived from species compatible with the host cell are used with these hosts. The vector ordinarily carries a replication site as well as marker sequences which are capable of providing phenotypic selection in transformed cells. For example, E. coli is typically transformed using pBR322, a plasmid derived from an E. coli species (Bolivar, et al., Gene 2: 95 (1977)). Plasmid pBR322 contains genes for ampicillin and tetracycline resistance and thus provides easy means for identifying transformed cells. The pBR322 plasmid, or other microbial plasmid must also contain or be modified to contain promoters and other control elements commonly used in recombinant DNA technology.

The desired coding sequence is inserted into an expression vector in the proper orientation to be transcribed from a promoter and ribosome binding site, both of which should be functional in the host cell in which the protein is to be expressed. An example of such an expression vector is a plasmid described in Belagaje et al., U.S. patent No. 5,304,493, the teachings of which are herein incorporated by reference. The gene encoding A-C-B proinsulin described in U.S. patent No. 5,304,493 can be removed from the plasmid pRB182 with restriction enzymes NdeI and BamHI. The genes encoding the protein of the present invention can be inserted into the plasmid backbone on a NdeI/BamHI restriction fragment cassette.

### 2.d. Procaryotic expression

In general, procaryotes are used for cloning of DNA sequences in constructing the vectors useful in the invention. For example, E. coli K12 strain 294 (ATCC No. 31446) is particularly useful. Other microbial strains which may be used include E. coli B and E. coli X1776 (ATCC No. 31537). These examples are illustrative rather than limiting.

Prokaryotes also are used for expression. The aforementioned strains, as well as E. coli W3110 (prototrophic, ATCC No. 27325), bacilli such as Bacillus subtilis, and other enterobacteriaceae such as Salmonella typhimurium or Serratia marcescans, and various pseudomonas species may be used. Promoters suitable for use with prokaryotic hosts include the β-lactamase (vector pGX2907 [ATCC 39344] contains the replicon and β-lactamase gene) and lactose promoter systems (Chang et al., Nature, 275:615 (1978); and Goeddel et al., Nature 281:544 (1979)), alkaline phosphatase, the tryptophan (trp) promoter system (vector pATH1 [ATCC 37695] is designed to facilitate expression of an open reading frame as a trpE fusion protein under control of the trp promoter) and hybrid promoters such as the tac promoter (isolatable from plasmid pDR540 ATCC-37282). However, other functional bacterial promoters, whose nucleotide sequences are generally known, enable one of skill in the art to ligate them to DNA encoding the protein using linkers or adaptors to supply any required restriction sites. Promoters for use in bacterial systems also will contain a Shine-Dalgarno sequence operably linked to the DNA encoding protein.

### 2.e. Eucaryotic expression

The protein may be recombinantly produced in eukaryotic expression systems. Preferred promoters controlling transcription in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. β-actin promoter. The early and late promoters of the SV40 virus are conveniently obtained as an SV40 restriction fragment which also contains the SV40 viral origin of replication. Fiers, et al., Nature, 273:113 (1978). The entire SV40 genome may be obtained from plasmid pBRSV, ATCC 45019. The immediate early promoter of the human cytomegalovirus may be obtained from plasmid pCMBβ (ATCC 77177). Of course, promoters from the host cell or related species also are useful herein.

Transcription of a DNA encoding the claimed protein by higher eukaryotes is increased by inserting an enhancer sequence into the vector. Enhancers are cis-acting elements of DNA, usually about 10-300 bp, that act on a promoter to increase its transcription. Enhancers are relatively orientation and position independent having been found 5' (Laimins, L. et al., PNAS 78:993 (1981)) and 3' (Lusky, M. L., et al., Mol. Cell Bio. 3:1108 (1983)) to the transcription unit, within an intron (Banerji, J. L. et al., Cell 33:729 (1983)) as well as within the coding sequence itself (Osborne, T. F., et al., Mol. Cell Bio. 4:1293 (1984)). Many enhancer sequences are now known from mammalian genes (globin, RSV, SV40, EMC, elastase, albumin, a-fetoprotein and insulin). Typically, however, one will use an enhancer from a eukaryotic cell virus. Examples include the SV40 late enhancer, the cytomegalovirus early promoter enhancer, the polyoma enhancer on the late side of the replication origin, and adenovirus enhancers.

Expression vectors used in eukaryotic host cells (yeast, fungi, insect, plant, animal, human or nucleated cells from other multicellular organisms) will also contain sequences necessary for the termination of transcription which may affect mRNA expression. These regions are transcribed as polyadenylated segments in the untranslated portion of the mRNA encoding protein. The 3' untranslated regions also include transcription termination sites.

Expression vectors may contain a selection gene, also termed a selectable marker. Examples of suitable selectable markers for mammalian cells are dihydrofolate reductase (DHFR, which may be derived from the BglII/HindIII restriction fragment of pJOD-10 [ATCC 68815]), thymidine kinase (herpes simplex virus thymidine kinase is contained on the BamHI fragment of vP-5 clone [ATCC 2028]) or neomycin (G418) resistance genes (obtainable from pNN414 yeast artificial chromosome vector [ATCC 37682]). When such selectable markers are successfully transferred into a mammalian host cell, the transfected mammalian host cell can survive if placed under selective pressure. There are two widely used distinct categories of selective regimes. The first category is based on a cell's metabolism and the use of a mutant cell line which lacks the ability to grow without a supplemented media. Two examples are: CHO DHFR⁻ cells (ATCC CRL-9096) and mouse LTK⁻ cells (L-M(TK-) ATCC CCL-2.3). These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in nonsupplemented media.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, Southern P. and Berg, P., J. Molec. Appl. Genet. 1: 327 (1982), mycophenolic acid, Mulligan, R. C. and Berg, P. Science 209:1422 (1980), or hygromycin, Sugden, B. et al., Mol Cell. Biol. 5:410-413 (1985). The three examples given above employ bacterial genes under eukaryotic control to convey resistance to the appropriate drug G418 or neomycin (geneticin), xgpt (mycophenolic acid) or hygromycin, respectively.

A preferred vector for eucaryotic expression is pRc/CMV. pRc/CMV is commercially available from Invitrogen Corporation, 3985 Sorrento Valley Blvd., San Diego, CA 92121. To confirm correct sequences in plasmids constructed, the ligation mixtures are used to transform E. coli K12 strain DH5a (ATCC 31446) and successful transformants selected by antibiotic resistance where appropriate. Plasmids from the transformants are prepared, analyzed by restriction and/or sequence by the method of Messing, et al., Nucleic Acids Res. 9:309 (1981).

Host cells may be transformed with the expression vectors of this invention and cultured in conventional nutrient media modified as is appropriate for inducing promoters, selecting transformants or amplifying genes. The culture conditions, such as temperature, pH and the like, are those previously used with the host cell selected for expression, and will be apparent to the ordinarily skilled artisan. The techniques of transforming cells with the aforementioned vectors are well known in the art and may be found in such general references as Maniatis, et al., Molecular Cloning: A Laboratory Manual, Cold Spring Harbor Press, Cold Spring Harbor Laboratory, Cold Spring Harbor, New York (1989), or Current Protocols in Molecular Biology (1989) and supplements.

Preferred suitable host cells for expressing the vectors encoding the claimed proteins in higher eukaryotes include: African green monkey kidney line cell line transformed by SV40 (COS-7, ATCC CRL-1651); transformed human primary embryonal kidney cell line 293, (Graham, F. L. et al., J. Gen Virol. 36:59-72 (1977), Virology 77:319-329, Virology 86:10-21); baby hamster kidney cells (BHK-21(C-13), ATCC CCL-10, Virology 16:147 (1962)); chinese hamster ovary cells CHO-DHFR⁻ (ATCC CRL-9096), mouse Sertoli cells (TM4, ATCC CRL-1715, Biol. Reprod. 23:243-250 (1980)); african green monkey kidney cells (VERO 76, ATCC CRL-1587); human cervical epitheloid carcinoma cells (HeLa, ATCC CCL-2); canine kidney cells (MDCK, ATCC CCL-34); buffalo rat liver cells (BRL 3A, ATCC CRL-1442); human diploid lung cells (WI-38, ATCC CCL-75); human hepatocellular carcinoma cells (Hep G2, ATCC HB-8065);and mouse mammary tumor cells (MMT 060562, ATCC CCL51).

### 2.f. Yeast expression

In addition to prokaryotes, eukaryotic microbes such as yeast cultures may also be used. Saccharomyces cerevisiae, or common baker's yeast is the most commonly used eukaryotic microorganism, although a number of other strains are commonly available. For expression in Saccharomyces, the plasmid YRp7, for example, (ATCC-40053, Stinchcomb, et al., Nature 282:39 (1979); Kingsman et al., Gene 7:141 (1979); Tschemper et al., Gene 10:157 (1980)) is commonly used. This plasmid already contains the trp gene which provides a selection marker for a mutant strain of yeast lacking the ability to grow in tryptophan, for example ATCC no. 44076 or PEP4-1 (Jones, Genetics 85:12 (1977)).

Suitable promoting sequences for use with yeast hosts include the promoters for 3-phosphoglycerate kinase (found on plasmid pAP12BD ATCC 53231 and described in U.S. Patent No. 4,935,350, June 19, 1990) or other glycolytic enzymes such as enolase (found on plasmid pAC1 ATCC 39532), glyceraldehyde-3-phosphate dehydrogenase (derived from plasmid pHcGAPC1 ATCC 57090, 57091), zymomonas mobilis (United States Patent No. 5,000,000 issued March 19, 1991), hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein (contained on plasmid vector pCL28XhoLHBPV ATCC 39475, United States Patent No. 4,840,896), glyceraldehyde 3-phosphate dehydrogenase, and enzymes responsible for maltose and galactose (GAL1 found on plasmid pRY121 ATCC 37658) utilization. Suitable vectors and promoters for use in yeast expression are further described in R. Hitzeman et al., European Patent Publication No. 73,657A. Yeast enhancers such as the UAS Gal from Saccharomyces cerevisiae (found in conjunction with the CYC1 promoter on plasmid YEpsec--hI1beta ATCC 67024), also are advantageously used with yeast promoters.

The following examples are presented to further illustrate the preparation of the claimed proteins. The scope of the present invention is not to be construed as merely consisting of the following examples.

### Example 1

A DNA sequence encoding the following protein sequence: Met Arg - SEQ ID NO: 1. is obtained using standard PCR methodology. A forward primer (5'-GG GG CAT ATG AGG GTA CCT ATC CAG AAA GTC CAG GAT GAC AC) (SEQ ID NO. 6) and a reverse primer (5'-GG GG GGATC CTA TTA GCA CCC GGG AGA CAG GTC CAG CTG CCA CAA CAT) (SEQ ID NO. 7) is used to amplify sequences from a human fat cell library (commercially available from CLONETECH). The PCR product is cloned into PCR-Script (available from STRATAGENE) and sequenced.

### Example 2

### Vector Construction

A plasmid containing the DNA sequence encoding the desired claimed protein is constructed to include NdeI and BamHI restriction sites. The plasmid carrying the cloned PCR product is digested with NdeI and BamHI restriction enzymes. The small ∼ 450bp fragment is gel-purified and ligated into the vector pRB182 from which the coding sequence for A-C-B proinsulin is deleted. The ligation products are transformed into E. coli DH10B (commercially available from GIBCO-BRL) and colonies growing on tryptone-yeast (DIFCO) plates supplemented with 10 µg/mL of tetracycline are analyzed. Plasmid DNA is isolated, digested with NdeI and BamHI and the resulting fragments are separated by agarose gel electrophoresis. Plasmids containing the expected ∼ 450bp NdeI to BamHI fragment are kept. E. coli B BL21 (DE3) (commercially available from NOVOGEN) are transformed with this second plasmid expression suitable for culture for protein production.

The techniques of transforming cells with the aforementioned vectors are well known in the art and may be found in such general references as Maniatis, et al. (1988) Molecular Cloning: A Laboratory Manual, Cold Spring Harbor Press, Cold Spring Harbor Laboratory, Cold Spring Harbor, New York or Current Protocols in Molecular Biology (1989) and supplements. The techniques involved in the transformation of E. coli cells used in the preferred practice of the invention as exemplified herein are well known in the art. The precise conditions under which the transformed E. coli cells are cultured is dependent on the nature of the E. coli host cell line and the expression or cloning vectors employed. For example, vectors which incorporate thermoinducible promoter-operator regions, such as the c1857 thermoinducible lambda-phage promoter-operator region, require a temperature shift from about 30 to about 40 degrees C. in the culture conditions so as to induce protein synthesis.

### Example 3

A DNA sequence encoding the protein of the Formula: 
(SEQ ID NO: 8)
Val Pro Ile Gln Lys Val Gln Asp Asp Thr Lys Thr Leu Ile Lys Thr
1               5                   10                  15
Ile Val Thr Arg Ile Asp Asp Ile Ser His Thr Gln Ser Val Ser Ser
            20                  25                  30
Lys Gln Lys Val Thr Gly Leu Asp Phe Ile Pro Gly Leu His Pro Ile
        35                  40                  45
Leu Thr Leu Ser Lys Met Asp Gln Thr Leu Ala Val Tyr Gln Gln Ile
    50                  55                  60
Leu Thr Ser Met Pro Ser Arg Asn Val Ile Gln Ile Ser Asn Asp Leu
65                  70                  75                  80
Glu Asn Leu Arg Asp Leu Leu His Val Leu Ala Phe Ser Lys Ser Cys
                85                  90                  95
His Leu Pro Trp Ala Ser Gly Leu Glu Thr Leu Asp Ser Leu Gly Gly
            100                 105                 110
Val Leu Glu Ala Ser Gly Tyr Ser Thr Glu Val Val Ala Leu Ser Arg
        115                 120                 125
Leu Gln Gly Ser Leu Gln Asp Met Leu Trp Gln Leu Asp Leu Ser Pro
    130                 135                 140
145
Gly Cys
 was assembled from chemically synthesized single stranded oligonucleotides to generate a double stranded DNA sequence. The oligonucleotides used to assemble this DNA sequence are as follows: 
(SEQ ID NO: 9)
TATGAGGGTACCTATCCAAAAAGTACAAGATGACACCAAAACACTGATAAAGACAATAGTC
ACAAG
 
(SEQ ID NO: 10)
GATAGATGATATCTCACACACACAGTCAGTCTCATCTAAACAGAAAGTCACAGGCTTGGAC
TTCATACCTGG
 
(SEQ ID NO: 11)
GCTGCACCCCATACTGACATTGTCTAAAATGGACCAGACACTGGCAGTCTATCAACAGATC
TTAACAAGTATGCCTT
 
(SEQ ID NO: 12)
CTAGAAGGCATACTTGTTAAGATCTGTTGATAGACTGC
 
(SEQ ID NO: 13)
CAGTGTCTGGTCCATTTTAGACAATGTCAGTATGGGGTGCAGCCCAGGTATGAAGTCCAAG
C
 
(SEQ ID NO: 14)
CTGTGACTTTCTGTTTAGATGAGACTGACTGTGTGTGAGATATCATCTATCCTTGTGAC
TATTGTCTTTATCAGTGTTTTG
 
(SEQ ID NO:  15)
GTGTCATCTTGTACTTTTTGGATAGGTACCCTCA
 
(SEQ ID NO: 16)
CTAGAAACGTGATACAAATATCTAACGACCTGGAGAACCTGCGGGATCTGCTGCACGTGCT
GGCCTTCTCTAAAAGTTGCCACTTGCCATGG
 
(SEQ ID NO: 17)
GCCAGTGGCCTGGAGACATTGGACAGTCTGGGGGAGTCCTGGAAGCCTCAGGCTATTCTA
CAGAGGTGGTGGC
 
(SEQ ID NO: 18)
CCTGAGCAGGCTGCAGGGGTCTCTGCAAGACATGCTGTGGCAGCTGGACCTGAGCCCCGGG
TGCTAATAG
 
(SEQ ID NO: 19)
GATCCTATTAGCACCCGGGGCTCAGGTCCAGCTGCCACAGCATGTCTTGCAGAGACC
 
(SEQ ID NO: 20)
CCTGCAGCCTGCTCAGGGCCACCACCTCTGTAGAATAGCCTGAGGCTTCCAGGACTCCC
 
(SEQ ID NO: 21)
CCCAGACTGTCCAATGTCTCCAGGCCACTGGCCCATGGCAAGTGGCAACTTTTAGAGAAGG
 
(SEQ ID NO: 22)
CCAGCACGTGCAGCAGATCCCGCAGGTTCTCCAGGTCGTTAGATATTTGTATCACGTTT
 Oligonucleotides 9 - 13 were used to generate an approximately 220 base-pair segment which extends from the NdeI site to the XbaI site at position 220 within the coding sequence. The oligonucleotides 14 - 22 were used to generate an approximately 240 base-pair segment which extends from the XbaI site to the BamHI site.

To assemble the 220 and 240 base-pair fragments, the respective oligonucleotides were mixed in equimolar amounts, usually at concentrations of about 1-2 picomoles per microliters. Prior to assembly, all but the oligonucleotides at the 5" -ends of the segment were phosphorylated in standard kinase buffer with T4 DNA kinase using the conditions specified by the supplier of the reagents. The mixtures were heated to 95°C and allowed to cool slowly to room temperature over a period of 1-2 hours to ensure proper annealing of the oligonucleotides. The oligonucleotides were then ligated to each other and into a cloning vector, PUC19 was used, but others are operable using T4 DNA ligase. The PUC19 buffers and conditions are those recommended by the supplier of the enzyme. The vector for the 220 base-pair fragment was digested with NdeI and XbaI, whereas the vector for the 240 base-pair fragment was digested with XbaI and BamHI prior to use. The ligation mixes were used to transform E. coli DH10B cells (commercially available from Gibco/BRL) and the transformed cells were plated on tryptone-yeast (TY) plates containing 100 µg/ml of ampicillin, X-gal and IPTG. Colonies which grow up overnight were grown in liquid TY medium with 100 µg/ml of ampicillin and are used for plasmid isolation and DNA sequence analysis. Plasmids with the correct sequence were kept for the assembly of the complete gene. This was accomplished by gel-purification of the 220 base-pair and the 240 base-pair fragments and ligation of these two fragments into PUC19 linearized with NdeI and BamHI. The ligation mix was transformed into E. coli DH10B cells and plated as described previously. Plasma DNA was isolated from the resulting transformants and digested with NdeI and BglII. The large vector fragment was gel-purified and ligated with a approximately 195 base-pair segment which was assembled as described previously from six chemically synthesized oligonucleotides as show below. 
(SEQ ID NO: 23)
TAT GCG GGT ACC GAT CCA GAA AGT TCA GGA CGA CAC CAA AAC CCT
GAT CAA AAC CAT CGT TAC
 
(SEQ ID NO: 24)
GCG TAT CAA CGA CAT CTC CCA CAC CCA GTC CGT GAG CTC CAA ACA
GAA GGT TAC CGG TCT GGA CTT CAT CCC GG
 
(SEQ ID NO: 25)
GTC TGC ACC CGA TCC TGA CCC TGT CCA AAA TGG ACC AGA CCC TGG
CTG TTT ACC AGC A
 
(SEQ ID NO: 26)
ATA CGC GTA ACG ATG GTT TTG ATC AGG GTT TTG GTG TCG TCC TGA
ACT TTC TGG ATC GGT ACC CGC A
 
(SEQ ID NO: 27)
TGC AGA CCC GGG ATG AAG TCC AGA CCG GTA ACC TTC TGT TTG GAG
CTC ACG GAC TGG GTG TGG GAG ATG TCG TTG
 
(SEQ ID NO: 28)
GAT CTG CTG GTA AAC AGC CAG GGT CTG GTC CAT TTT GGA CAG GGT
CAG GAT CGG G
 The ligation was transformed into E. coli cells as described previously. The DNA from the resulting transformants was isolated and the sequence was verified by DNA sequence analysis. The plasmid with the correct sequence was digested with NdeI and BamHI and approximately 450 base-pair insert was recloned into an expression vector.

The protein was expressed in E. coli, isolated and was folded either by dilution into PBS or by dilution into 8M urea (both containing 5 mM cysteine) and exhaustive dialysis against PBS. Following final purification of the proteins by size exclusion chromatography the proteins were concentrated to 3-3.5 mg/mL in PBS. Amino acid composition was confirmed.

### Example 4

The protein of SEQ ID NO: 2 with a Met Arg leader sequence was expressed in E. coli. Granules were solubilized in 8M urea containing 5mM cysteine. The protein was purified by anion exchange chromatography and folded by dilution into 8M urea (containing 5 mM cysteine) and exhaustive dialysis against PBS by techniques analogous to the previous Examples. The Met Arg leader sequence was cleaved by the addition of 6-10 milliunits dDAP per mg of protein. The conversion reaction was allowed to proceed for 2-8 hours at room temperature. The progress of the reaction was monitored by high performance reversed phase chromatography. The reaction was terminated by adjusting the pH to 8 with NaOH. The des(Met-Arg) protein was further purified by cation exchange in the presence of 7-8 M urea and dialyzed into PBS. Following final purification of the proteins by size exclusion chromatography the proteins were concentrated to 3-3.5 mg/mL in PBS.

In the preferred embodiment of the invention E. coli K12 RV308 cells are employed as host cells but numerous other cell lines are available such as, but not limited to, E. coli K12 L201, L687, L693, L507, L640, L641, L695, L814 (E. coli B). The transformed host cells are then plated on appropriate media under the selective pressure of the antibiotic corresponding to the resistance gene present on the expression plasmid. The cultures are then incubated for a time and temperature appropriate to the host cell line employed.

Proteins which are expressed in high-level bacterial expression systems characteristically aggregate in granules or inclusion bodies which contain high levels of the overexpressed protein. Kreuger et al., in Protein Folding, Gierasch and King, eds., pgs 136-142 (1990), American Association for the Advancement of Science Publication No. 89-18S, Washington, D.C. Such protein aggregates must be solubilized to provide further purification and isolation of the desired protein product. Id. A variety of techniques using strongly denaturing solutions such as guanidinium-HCl and/or weakly denaturing solutions such as dithiothreitol (DTT) are used to solubilize the proteins. Gradual removal of the denaturing agents (often by dialysis) in a solution allows the denatured protein to assume its native conformation. The particular conditions for denaturation and folding are determined by the particular protein expression system and/or the protein in question.

Preferably, the present proteins are expressed as Met-Arg-SEQ ID NO: 1 so that the expressed proteins may be readily converted to the claimed protein with Cathepsin C. The purification of proteins is by techniques known in the art and includes reverse phase chromatography, affinity chromatography, and size exclusion. Significantly, the proteins with the leader sequence attached were found to be active. Accordinlgy, the present invention provides proteins of the Formula I with a Met-R₁ leader sequence, wherein R₁ is any naturally occurring amino acid except Pro, preferably R₁ is Arg, Asp, or Tyr and most preferably, arginine.

The claimed proteins contain two cysteine residues. Thus, a di-sulfide bond may be formed to stabilize the protein. The present invention includes proteins of the Formula (I) wherein the Cys at position 96 of SEQ ID NO: 1 is crosslinked to Cys at position 146 of SEQ ID NO: 1 as well as those proteins without such di-sulfide bonds. In addition the proteins of the present invention may exist, particularly when formulated, as dimers, trimers, tetramers, and other multimers. Such multimers are included within the scope of the present invention.

The present invention provides a method for treating obesity. The method comprises administering to the organism an effective amount of anti-obesity protein in a dose between about 1 and 1000 µg/kg. A preferred dose is from about 10 to 100 µg/kg of active compound. A typical daily dose for an adult human is from about 0.5 to 100 mg. In practicing this method, compounds of the Formula (I) can be administered in a single daily dose or in multiple doses per day. The treatment regime may require administration over extended periods of time. The amount per administered dose or the total amount administered will be determined by the physician and depend on such factors as the nature and severity of the disease, the age and general health of the patient and the tolerance of the patient to the compound.

The instant invention further provides pharmaceutical formulations comprising compounds of the Formula (I). The proteins, preferably in the form of a pharmaceutically acceptable salt, can be formulated for parenteral administration for the therapeutic or prophylactic treatment of obesity.
For example, compounds of the Formula (I) can be admixed with conventional pharmaceutical carriers and excipients. The compositions comprising claimed proteins contain from about 0.1 to 90% by weight of the active protein, preferably in a soluble form, and more generally from about 10 to 30%. Furthermore, the present proteins may be administered alone or in combination with other anti-obesity agents or agents useful in treating diabetes.

For intravenous (IV) use, the protein is administered in commonly used intravenous fluid(s) and administered by infusion. Such fluids, for example, physiological saline, Ringer's solution or 5% dextrose solution can be used.

For intramuscular preparations, a sterile formulation, preferably a suitable soluble salt form of a protein of the Formula (I) , for example the hydrochloride salt, can be dissolved and administered in a pharmaceutical diluent such as pyrogen-free water (distilled), physiological saline or 5% glucose solution. A suitable insoluble form of the compound may be prepared and administered as a suspension in an aqueous base or a pharmaceutically acceptable oil base, e.g. an ester of a long chain fatty acid such as ethyl oleate.

The ability of the present compounds to treat obesity is demonstrated *in vivo* as follows:

### Biological Testing for Anti-obesity proteins

Parabiotic experiments suggest that a protein is released by peripheral adipose tissue and that the protein is able to control body weight gain in normal, as well as obese mice. Therefore, the most closely related biological test is to inject the test article by any of several routes of administration (e.g. i.v., s.c., i.p., or by minipump or cannula) and then to monitor food and water consumption, body weight gain, plasma chemistry or hormones (glucose, insulin, ACTH, corticosterone, GH, T4) over various time periods.

Suitable test animals include normal mice (ICR, etc.) and obese mice (*ob/ob,* Avy/a, KK-Ay, tubby, fat). The *ob/ob* mouse model of obesity and diabetes is generally accepted in the art as being indicative of the obesity condition. Controls for non-specific effects for these injections are done using vehicle with or without the active agent of similar composition in the same animal monitoring the same parameters or the active agent itself in animals that are thought to lack the receptor (db/db mice, fa/fa or cp/cp rats). Proteins demonstrating activity in these models will demonstrate similar activity in other mammals, particularly humans.

Since the target tissue is expected to be the hypothalamus where food intake and lipogenic state are regulated, a similar model is to inject the test article directly into the brain (e.g. i.c.v. injection via lateral or third ventricles, or directly into specific hypothalamic nuclei (e.g. arcuate, paraventricular, perifornical nuclei). The same parameters as above could be measured, or the release of neurotransmitters that are known to regulate feeding or metabolism could be monitored (e.g. NPY, galanin, norepinephrine, dopamine, β-endorphin release).

Similar studies are accomplished *in vitro* using isolated hypothalamic tissue in a perifusion or tissue bath system. In this situation, the release of neurotransmitters or electrophysiological changes is monitored.

The compounds are active in at least one of the above biological tests and are anti-obesity agents. As such, they are useful in treating obesity and those disorders implicated by obesity. Representative proteins outlined in Table 1 were prepared in accordance with the teachings and examples provided herein. The description of the protein in Table 1 represents a protein of SEQ ID NO: 29 wherein the amino acid designated in the description is replaced as provided in Formula (I). For example, Asp22 designates a protein of SEQ ID NO: 29 wherein Asn at position 22 is replaced with Asp. The designation Met Arg - indicates that the protein was prepared and tested with a Met Arg leader sequence attached. Amino acid sequences of the proteins of Table 1 were confirmed by mass spectroscopy and/or amino acid analysis.

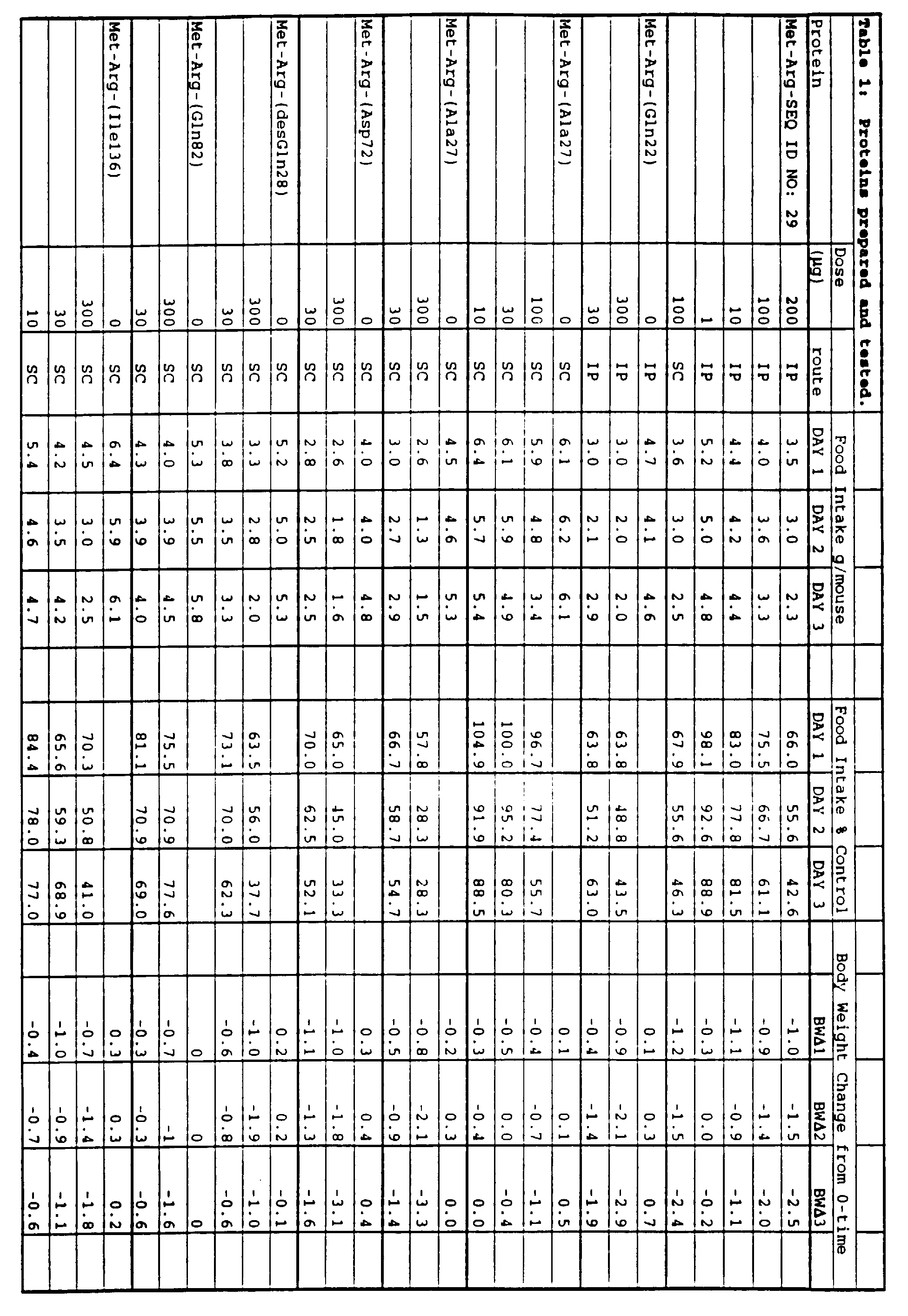

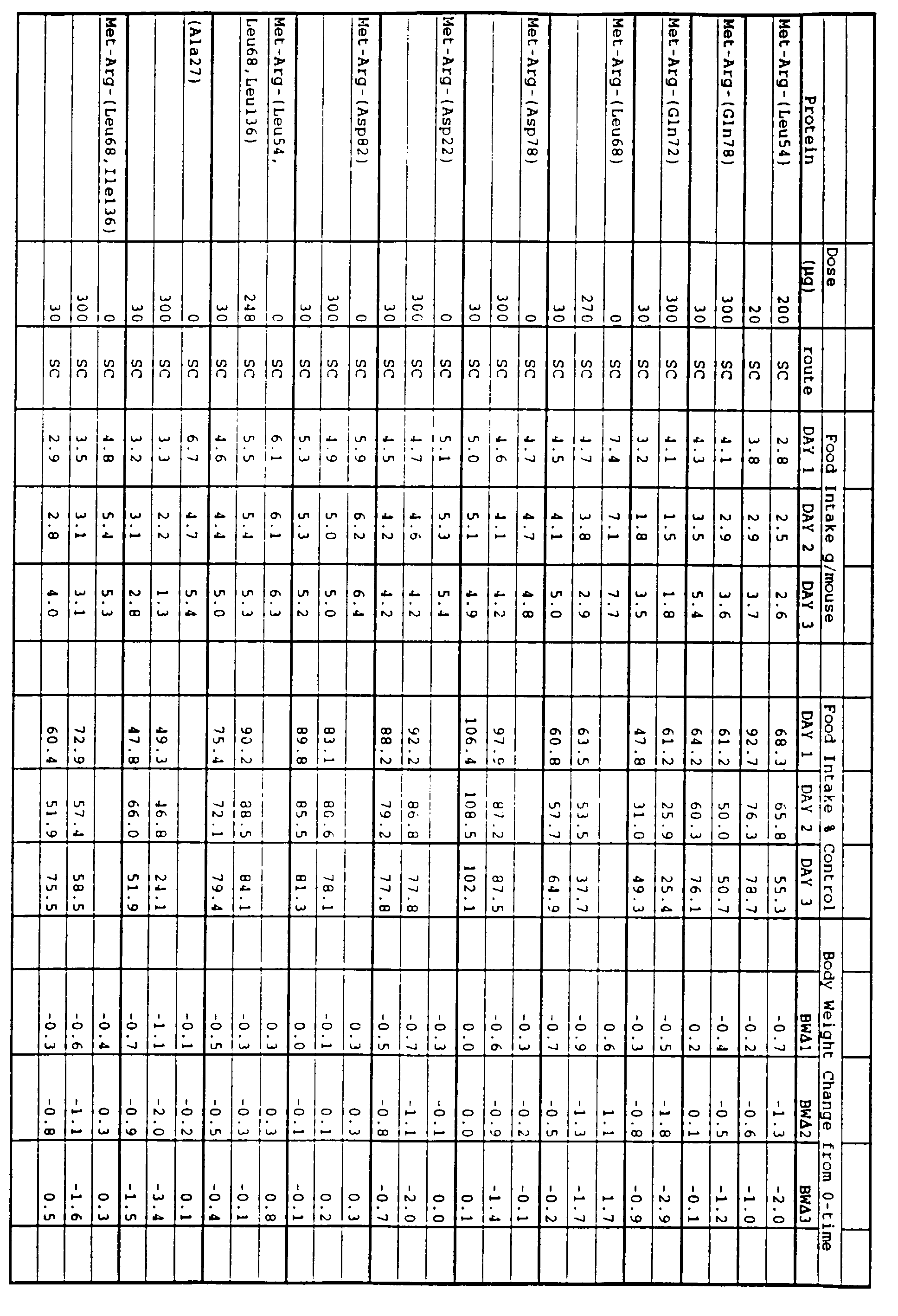

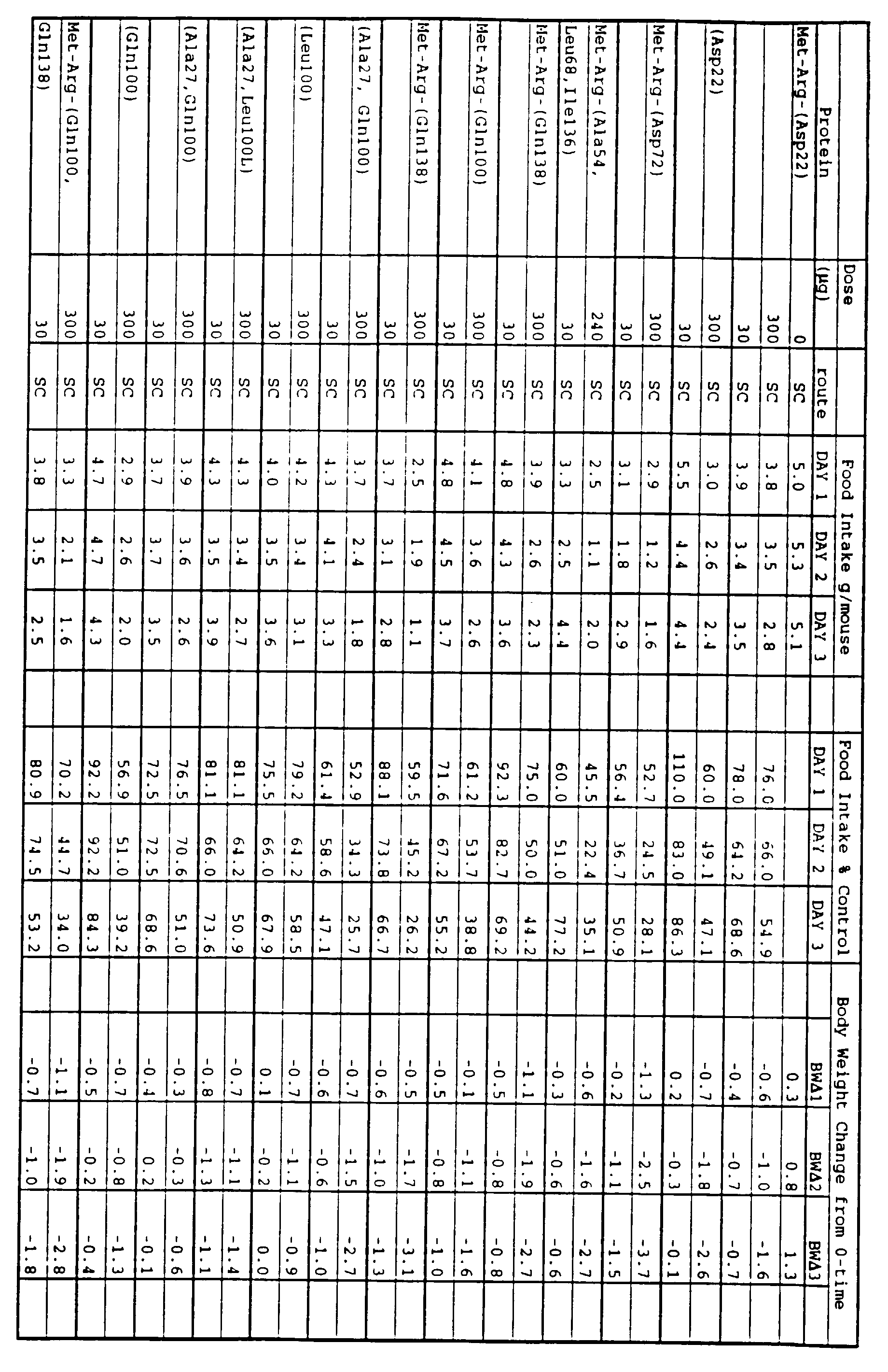

(SEQ ID NO: 29)
5                    10                    15

Val Pro Ile Gln Lys Val Gln Asp Asp Thr Lys Thr Leu Ile Lys Thr

20 25 30
Ile Val Thr Arg Ile Asn Asp Ile Ser His Thr Gln Ser Val Ser Ser

35 40 45
Lys Gln Lys Val Thr Gly Leu Asp Phe Ile Pro Gly Leu His Pro Ile

50 55 60
Leu Thr Leu Ser Lys Met Asp Gln Thr Leu Ala Val Tyr Gln Gln Ile

65 70 75 80
Leu Thr Ser Met Pro Ser Arg Asn Val Ile Gln Ile Ser Asn Asp Leu

85 90 95
Glu Asn Leu Arg Asp Leu Leu His Val Leu Ala Phe Ser Lys Ser Cys

100 105 110
His Leu Pro Trp Ala Ser Gly Leu Glu Thr Leu Asp Ser Leu Gly Gly

115 120 125
Val Leu Glu Ala Ser Gly Tyr Ser Thr Glu Val Val Ala Leu Ser Arg

130 135 140
Leu Gln Gly Ser Leu Gln Asp Met Leu Trp Gln Leu Asp Leu Ser Pro

145
Gly Cys

The proteins are not only useful as therapeutic agents; one skilled in the art recognizes that the proteins are useful in the production of antibodies for diagnostic use and, as proteins, are useful as feed additives for animals. Furthermore, the compounds are useful for controlling weight for cosmetic purposes in mammals. A cosmetic purpose seeks to control the weight of a mammal to improve bodily appearance. The mammal is not necessarily obese. Such cosmetic use forms part of the present invention.

The principles, preferred embodiments and modes of operation of the present invention have been described in the foregoing specification. The invention that is intended to be protected herein, however, is not to be construed as limited to the particular forms disclosed, since they are to be regarded as illustrative rather than restrictive. Variations and changes may be made by those skilled in the art without departing from the spirit of invention.

## Claims

1. A protein of the Formula (I) : 
(SEQ ID NO: 1)
5 10 15
Val Pro Ile Xaa Lys Val Xaa Asp Asp Thr Lys Thr Leu Ile Lys Thr

20 25 30
Ile Val Thr Arg Ile Xaa Asp Ile Ser His Xaa Xaa Ser Val Ser Ser

35 40 45
Lys Xaa Lys Val Thr Gly Leu Asp Phe Ile Pro Gly Leu His Pro Ile

50 55 60
Leu Thr Leu Ser Lys Xaa Asp Xaa Thr Leu Ala Val Tyr Xaa Xaa Ile

65 70 75 80
Leu Thr Ser Xaa Pro Ser Arg Xaa Val Ile Xaa Ile Ser Xaa Asp Leu

85 90 95
Glu Xaa Leu Arg Asp Leu Leu His Val Leu Ala Phe Ser Lys Ser Cys

100 105 110
His Leu Pro Xaa Ala Ser Gly Leu Glu Thr Leu Xaa Ser Leu Gly Gly

115 120 125
Val Leu Glu Ala Ser Gly Tyr Ser Thr Glu Val Val Ala Leu Ser Arg

130 135 140
Leu Xaa Gly Ser Leu Xaa Asp Xaa Leu Xaa Xaa Leu Asp Leu Ser Pro

145
Gly Cys

(I)
 wherein:
Xaa at position 4 is Gln or Glu;
Xaa at position 7 is Gln or Glu;
Xaa at position 22 is Gln, Asn, or Asp;
Xaa at position 27 is Thr or Ala;
Xaa at position 28 is Gln, Glu, or absent;
Xaa at position 34 is Gln or Glu;
Xaa at position 54 is Met, methionine sulfoxide, Leu, Ile, Val, Ala, or Gly;
Xaa at position 56 is Gln or Glu;
Xaa at position 62 is Gln or Glu;
Xaa at position 63 is Gln or Glu;
Xaa at position 68 is Met, methionine sulfoxide, Leu, Ile, Val, Ala, or Gly;
Xaa at position 72 is Gln, Asn, or Asp;
Xaa at position 75 is Gln or Glu;
Xaa at position 78 is Gln, Asn, or Asp;
Xaa at position 82 is Gln, Asn, or Asp;
Xaa at position 100 is Glu, Trp, Phe, Ile, Val, or Leu;
Xaa at position 108 is Asp or Glu;
Xaa at position 130 is Gln or Glu;
Xaa at position 134 is Gln or Glu;
Xaa at position 136 is Met, methionine sulfoxide, Leu, Ile, Val, Ala, or Gly;
Xaa at position 138 is Gln, Trp, Tyr, Phe, Ile, Val, or Leu;
Xaa at position 139 is Gln or Glu;
or a pharmaceutically acceptable salt thereof;
with the exception of a protein wherein:
Xaa at position 4 is Gln;
Xaa at position 7 is Gln;
Xaa at position 22 is Asn;
Xaa at position 27 is Thr;
Xaa at position 28 is Gln or absent;
Xaa at position 34 is Gln;
Xaa at position 54 is Met;
Xaa at position 56 is Gln;
Xaa at position 62 is Gln;
Xaa at position 63 is Gln;
Xaa at position 68 is Met;
Xaa at position 72 is Asn;
Xaa at position 75 is Gln;
Xaa at position 78 is Asn;
Xaa at position 82 is Asn;
Xaa at position 100 is Trp;
Xaa at position 108 is Asp;
Xaa at position 130 is Gln;
Xaa at position 134 is Gln;
Xaa at position 136 is Met;
Xaa at position 138 is Trp; and
Xaa at position 139 is Gln.

2. A protein of the Formula (Ia) : 
(SEQ ID NO: 30)
                5                   10                  15
Val Pro Ile Xaa Lys Val Xaa Asp Asp Thr Lys Thr Leu Ile Lys Thr

            20                  25                  30
Ile Val Thr Arg Ile Xaa Asp Ile Ser His Xaa Xaa Ser Val Ser Ser

        35                  40                  45
Lys Xaa Lys Val Thr Gly Leu Asp Phe Ile Pro Gly Leu His Pro Ile

    50                  55                  60
Leu Thr Leu Ser Lys Xaa Asp Xaa Thr Leu Ala Val Tyr Xaa Xaa Ile

65                  70                  75                  80
Leu Thr Ser Xaa Pro Ser Arg Xaa Val Ile Xaa Ile Ser Xaa Asp Leu

                85                  90                  95
Glu Xaa Leu Arg Asp Leu Leu His Val Leu Ala Phe Ser Lys Ser Cys

            100                 105                 110
His Leu Pro Trp Ala Ser Gly Leu Glu Thr Leu Xaa Ser Leu Gly Gly

        115                 120                 125
Val Leu Glu Ala Ser Gly Tyr Ser Thr Glu Val Val Ala Leu Ser Arg

    130                 135                 140
Leu Xaa Gly Ser Leu Xaa Asp Xaa Leu Trp Xaa Leu Asp Leu Ser Pro

145
Gly Cys

(Ia)
 wherein:
Xaa at position 4 is Gln or Glu;
Xaa at position 7 is Gln or Glu;
Xaa at position 22 is Gln, Asn, or Asp;
Xaa at position 27 is Thr or Ala;
Xaa at position 28 is Gln, Glu, or absent;
Xaa at position 34 is Gln or Glu;
Xaa at position 54 is Met, methionine suifoxide, Leu, Ile, Val, Ala, or Gly;
Xaa at position 56 is Gln or Glu;
Xaa at position 62 is Gln or Glu;
Xaa at position 63 is Gln or Glu;
Xaa at position 68 is Met, methionine sulfoxide, Leu, Ile, Val, Ala, or Gly;
Xaa at position 72 is Gln, Asn, or Asp;
Xaa at position 75 is Gln or Glu;
Xaa at position 78 is Gln, Asn, or Asp;
Xaa at position 82 is Gln, Asn, or Asp;
Xaa at position 108 is Asp or Glu;
Xaa at position 130 is Gln or Glu;
Xaa at position 134 is Gln or Glu;
Xaa at position 136 is Met, methionine sulfoxide, Leu, Ile, Val, Ala, or Gly;
Xaa at position 139 is Gln or Glu;
or a pharmaceutically acceptable salt thereof;
with the exception of a protein wherein:
Xaa at position 4 is Gln;
Xaa at position 7 is Gln;
Xaa at position 22 is Asn;
Xaa at position 27 is Thr;
Xaa at position 28 is Gln;
Xaa at position 34 is Gln;
Xaa at position 54 is Met;
Xaa at position 56 is Gln;
Xaa at position 62 is Gln;
Xaa at position 63 is Gln;
Xaa at position 68 is Met;
Xaa at position 72 is Asn;
Xaa at position 75 is Gln;
Xaa at position 78 is Asn;
Xaa at position 82 is Asn;
Xaa at position 108 is Asp;
Xaa at position 130 is Gln;
Xaa at position 134 is Gln;
Xaa at position 136 is Met; and
Xaa at position 139 is Gln.

3. A protein of Claim 2, which is 
(SEQ ID NO: 2)
5 10 15
Val Pro Ile Gln Lys Val Gln Asp Asp Thr Lys Thr Leu Ile Lys Thr
20 25 30
Ile Val Thr Arg Ile Asp Asp Ile Ser His Thr Gln Ser Val Ser Ser
 
35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Val Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Trp Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys    ;
 wherein the Cys at position 96 is di-sulfide bonded to the Cys at position 146;
or a pharmaceutically acceptable salt thereof.

4. A protein of Claim 1, 2, or 3, further comprising a leader sequence of the formula: Met-R₁- ; wherein R₁ is any naturally occurring amino acid except Pro.

5. A protein of Claim 4, wherein R₁ is Arg.

6. A pharmaceutical formulation, which comprises a protein of Claim 1, 2, or 3 together with one or more pharmaceutical acceptable diluents, carriers or excipients therefor.

7. A process of making a protein of Claim 1, 2 or 3, which comprises:
(a) transforming a host cell with DNA that encodes the protein of Claim 1, 2 or 3, said protein having an optional leader sequence;
(b) culturing the host cell and isolating the protein encoded in step (a); and, optionally,
(c) cleaving enzymatically the leader sequence to produce the protein of Claim 1, 2 or 3.

8. The process of Claim 7, wherein the leader sequence is Met-R₁- ; wherein R₁ is any naturally occurring amino acid except Pro.

9. The process of Claim 8, wherein the leader sequence is Met-Arg-.

10. A protein as claimed in Claim 1, 2, or 3, for use in treating obesity.

11. A process for preparing a protein of Claim 1, 2, or 3 substantially as hereinbefore described with reference to any one of the examples.

12. A protein substantially as hereinbefore described with reference to any of the examples.


SEQUENCE LISTING

(1) GENERAL INFORMATION:

(i) APPLICANT:
(A) NAME: Eli Lilly and Company
(B) STREET: Lilly Corporate Center
(C) CITY: Indianapolis
(D) STATE: Indiana
(E) COUNTRY: United States
(F) POSTAL CODE (ZIP): 46285

(ii) TITLE OF INVENTION: Anti-obesity proteins

(iii) NUMBER OF SEQUENCES: 29

(iv) CORRESPONDENCE ADDRESS:
(A) ADRESSEE: K. G. Tapping
(B) STREET: Erl Wood Manor
(C) CITY: Windlesham
(D) STATE: Surrey
(E) COUNTRY: United Kingdom
(F) ZIP: GU20 6PH

(v) COMPUTER READABLE FORM:
(A) MEDIUM TYPE: Floppy disk
(B) COMPUTER: Macintosh
(C) OPERATING SYSTEM: Macintosh 7.0
(D) SOFTWARE: Microsoft Word 5.1

(2) INFORMATION FOR SEQ ID NO: 1:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION:1
(D) OTHER INFORMATION:/note= "Xaa at position 4 is Gln or
Glu;"

(ix) FEATURE:
(A) NAME/KEY: Protein
(B) LOCATION:7
(D) OTHER INFORMATION:/note= "Xaa at position 7 is Gln or
Glu;"

(ix) FEATURE:
(A) NAME/KEY: Protein
(B) LOCATION:22
(D) OTHER INFORMATION:/note= "Xaa at position 22 is Gln,
Asn, or Asp;"

(ix) FEATURE:
(A) NAME/KEY: Protein
(B) LOCATION:27
(D) OTHER INFORMATION:/note= "Xaa at position 27 is Thr
or Ala;"

(ix) FEATURE:
(A) NAME/KEY: Protein
(B) LOCATION:28
(D) OTHER INFORMATION:/note= "Xaa at position 28 is Gln,
Glu, or absent;"

(ix) FEATURE:
(A) NAME/KEY: Protein
(B) LOCATION:34
(D) OTHER INFORMATION:/note= "Xaa at position 34 is Gln
or Glu;"

(ix) FEATURE:
(A) NAME/KEY: Protein
(B) LOCATION:54
(D) OTHER INFORMATION:/note= "Xaa at position 54 is Met,
methionine sulfoxide, Leu, Ile, Val, Ala, or Gly;"

(ix) FEATURE:
(A) NAME/KEY: Protein
(B) LOCATION:56
(D) OTHER INFORMATION:/note= "Xaa at position 56 is Gln
or Glu;"

(ix) FEATURE:
(A) NAME/KEY: Protein
(B) LOCATION:62
(D) OTHER INFORMATION:/note= "Xaa at position 62 is Gln
or Glu;"

(ix) FEATURE:
(A) NAME/KEY: Protein
(B) LOCATION:63
(D) OTHER INFORMATION:/note= "Xaa at position 63 is Gln
or Glu;"

(ix) FEATURE:
(A) NAME/KEY: Protein
(B) LOCATION:68
(D) OTHER INFORMATION:/note= "Xaa at position 68 is Met,
methionine sulfoxide, Leu, Ile, Val, Ala, or Gly;"

(ix) FEATURE:
(A) NAME/KEY: Protein
(B) LOCATION:72
(D) OTHER INFORMATION:/note= "Xaa at position 72 is Gln,
Asn, or Asp;"

(ix) FEATURE:
(A) NAME/KEY: Protein
(B) LOCATION:75
(D) OTHER INFORMATION:/note= "Xaa at position 75 is Gln
or Glu;"

(ix) FEATURE:
(A) NAME/KEY: Protein
(B) LOCATION:78
(D) OTHER INFORMATION:/note= "Xaa at position 78 is Gln,
Asn, or Asp;"

(ix) FEATURE:
(A) NAME/KEY: Protein
(B) LOCATION:82
(D) OTHER INFORMATION:/note= "Xaa at position 82 is Gln,
Asn, or Asp;"

(ix) FEATURE:
(A) NAME/KEY: Protein
(B) LOCATION:100
(D) OTHER INFORMATION:/note= "Xaa at position 100 is Glu,
Trp, Phe, Ile, Val, or Leu;"

(ix) FEATURE:
(A) NAME/KEY: Protein
(B) LOCATION:108
(D) OTHER INFORMATION:/note= "Xaa at position 108 is Asp
or Glu;"

(ix) FEATURE:
(A) NAME/KEY: Protein
(B) LOCATION:130
(D) OTHER INFORMATION:/note= "Xaa at position 130 is Gln
or Glu;"

(ix) FEATURE:
(A) NAME/KEY: Protein
(B) LOCATION:134
(D) OTHER INFORMATION:/note= "Xaa at position 134 is Gln
or Glu;"

(ix) FEATURE:
(A) NAME/KEY: Protein
(B) LOCATION:136
(D) OTHER INFORMATION:/note= "Xaa at position 136 is Met,
methionine sulfoxide, Leu, Ile, Val, Ala, or Gly;"

(ix) FEATURE:
(A) NAME/KEY: Protein
(B) LOCATION:138
(D) OTHER INFORMATION:/note= "Xaa at position 138 is Gln, Trp,
Tyr, Phe, Ile, Val, or Leu;"

(ix) FEATURE:
(A) NAME/KEY: Protein
(B) LOCATION:139
(D) OTHER INFORMATION:/note= "Xaa at position 139 is
Gln or Glu;

(xi) SEQUENCE DESCRIPTION: SEQ ID NO: 1:

Val Pro Ile Xaa Lys Val Xaa Asp Asp Thr Lys Thr Leu Ile Lys Thr
                5                   10                  15

Ile Val Thr Arg Ile Xaa Asp Ile Ser His Xaa Xaa Ser Val Ser Ser
            20                  25                  30

Lys Xaa Lys Val Thr Gly Leu Asp Phe Ile Pro Gly Leu His Pro Ile
        35                  40                  45

Leu Thr Leu Ser Lys Xaa Asp Xaa Thr Leu Ala Val Tyr Xaa Xaa Ile
    50                  55                  60

Leu Thr Ser Xaa Pro Ser Arg Xaa Val Ile Xaa Ile Ser Xaa Asp Leu
65                  70                  75                  80

Glu Xaa Leu Arg Asp Leu Leu His Val Leu Ala Phe Ser Lys Ser Cys
                85                  90                  95

His Leu Pro Xaa Ala Ser Gly Leu Glu Thr Leu Xaa Ser Leu Gly Gly
            100                 105                 110

Val Leu Glu Ala Ser Gly Tyr Ser Thr Glu Val Val Ala Leu Ser Arg
        115                 120                 125

Leu Xaa Gly Ser Leu Xaa Asp Xaa Leu Xaa Xaa Leu Asp Leu Ser Pro
    130                 135                 140

Gly Cys
145

(2) INFORMATION FOR SEQ ID NO: 2:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 2:

Val Pro Ile Gln Lys Val Gln Asp Asp Thr Lys Thr Leu Ile Lys Thr
                5                   10                  15

Ile Val Thr Arg Ile Asp Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO: 3:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 3:

Val Pro Ile Gln Lys Val Gln Asp Asp Thr Lys Thr Leu Ile Lys Thr
                5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Gln Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO: 4:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 4:

Val Pro Ile Gln Lys Val Gln Asp Asp Thr Lys Thr Leu Ile Lys Thr
                5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

(2) INFORMATION FOR SEQ ID NO: 5:

    (i) SEQUENCE CHARACTERISTICS:
        (A) LENGTH: 146 amino acids
        (B) TYPE: amino acid
        (C) STRANDEDNESS: single
        (D) TOPOLOGY: linear

    (ii) MOLECULE TYPE: protein

    (xi) SEQUENCE DESCRIPTION: SEQ ID NO: 5:

Val Pro Ile Gln Lys Val Gln Asp Asp Thr Lys Thr Leu Ile Lys Thr
                5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Gln Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Gln Gln Leu Asp Leu Ser Pro
    130                 135                 140

Gly Cys
145

(2) INFORMATION FOR SEQ ID NO: 6:

(i) SEQUENCE CHARACTERISTICS:
(A) LENGTH: 48 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 6:

GGGGCATATG AGGGTACCTA TCCAGAAAGT CCAGGATGAC AC
42

(2) INFORMATION FOR SEQ ID NO: 7:

(i) SEQUENCE CHARACTERISTICS:
(A) LENGTH: 48 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 7:

GGGGGGATCC TATTAGCACC CGGGAGACAG GTCCAGCTGC
CACAACAT 48

(2) INFORMATION FOR SEQ ID NO: 8:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 8:

Val Pro Ile Gln Lys Val Gln Asp Asp Thr Lys Thr Leu Ile Lys Thr
                5                   10                  15

Ile Val Thr Arg Ile Asp Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO: 9:

(i) SEQUENCE CHARACTERISTICS:
(A) LENGTH: 66 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 9:

TATGAGGGTA CCTATCCAAA AAGTACAAGA TGACACCAAA
ACACTGATAA AGACAATAGT 60

CACAAG 66

(2) INFORMATION FOR SEQ ID NO: 10:

(i) SEQUENCE CHARACTERISTICS:
(A) LENGTH: 72 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 10:

GATAGATGAT ATCTCACACA CACAGTCAGT CTCATCTAAA
CAGAAAGTCA CAGGCTTGGA 60

CTTCATACCT GG 72

(2) INFORMATION FOR SEQ ID NO: 11:

(i) SEQUENCE CHARACTERISTICS:
(A) LENGTH: 77 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 11:

GCTGCACCCC ATACTGACAT TGTCTAAAAT GGACCAGACA
CTGGCAGTCT ATCAACAGAT 60

CTTAACAAGT ATGCCTT 77

(2) INFORMATION FOR SEQ ID NO: 12:

(i) SEQUENCE CHARACTERISTICS:
(A) LENGTH: 38 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 12:

CTAGAAGGCA TACTTGTTAA GATCTGTTGA TAGACTGC
38

(2) INFORMATION FOR SEQ ID NO: 13:

(i) SEQUENCE CHARACTERISTICS:
(A) LENGTH: 62 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 13:

CAGTGTCTGG TCCATTTTAG ACAATGTCAG TATGGGGTGC
AGCCCAGGTA TGAAGTCCAA 60

GC 62

(2) INFORMATION FOR SEQ ID NO: 14:

(i) SEQUENCE CHARACTERISTICS:
(A) LENGTH: 83 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 14:

CTGTGACTTT CTGTTTAGAT GAGACTGACT GTGTGTGTGA
GATATCATCT ATCCTTGTGA 60

CTATTGTCTT TATCAGTGTT TTG 83

(2) INFORMATION FOR SEQ ID NO: 15:

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 15:

GTGTCATCTT GTACTTTTTG GATAGGTACC CTCA 34

(2) INFORMATION FOR SEQ ID NO: 16:

(i) SEQUENCE CHARACTERISTICS:
(A) LENGTH: 92 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 16:

CTAGAAACGT GATACAAATA TCTAACGACC TGGAGAACCT
GCGGGATCTG CTGCACGTGC 60

TGGCCTTCTC TAAAAGTTGC CACTTGCCAT GG 92

(2) INFORMATION FOR SEQ ID NO: 17:

(i) SEQUENCE CHARACTERISTICS:
(A) LENGTH: 74 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 17:

GCCAGTGGCC TGGAGACATT GGACAGTCTG GGGGGAGTCC
TGGAAGCCTC AGGCTATTCT 60

ACAGAGGTGG TGGC 74

(2) INFORMATION FOR SEQ ID NO: 18:

(i) SEQUENCE CHARACTERISTICS:
(A) LENGTH: 70 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 18:

CCTGAGCAGG CTGCAGGGGT CTCTGCAAGA CATGCTGTGG
CAGCTGGACC TGAGCCCCGG 60

GTGCTAATAG 70

(2) INFORMATION FOR SEQ ID NO: 19:

(i) SEQUENCE CHARACTERISTICS:
(A) LENGTH: 57 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 19:

GATCCTATTA GCACCCGGGG CTCAGGTCCA GCTGCCACAG
CATGTCTTGC AGAGACC 57

(2) INFORMATION FOR SEQ ID NO: 20:

(i) SEQUENCE CHARACTERISTICS:
(A) LENGTH: 59 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 20:

CCTGCAGCCT GCTCAGGGCC ACCACCTCTG TAGAATAGCC
TGAGGCTTCC AGGACTCCC 59

(2) INFORMATION FOR SEQ ID NO: 21:

(i) SEQUENCE CHARACTERISTICS:
(A) LENGTH: 61 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 21:

CCCAGACTGT CCAATGTCTC CAGGCCACTG GCCCATGGCA
AGTGGCAACT TTTAGAGAAG 60

G 61

(2) INFORMATION FOR SEQ ID NO: 22:

(i) SEQUENCE CHARACTERISTICS:
(A) LENGTH: 59 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 22:

CCAGCACGTG CAGCAGATCC CGCAGGTTCT CCAGGTCGTT
AGATATTTGT ATCACGTTT 59

(2) INFORMATION FOR SEQ ID NO: 23:

(i) SEQUENCE CHARACTERISTICS:
(A) LENGTH: 63 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 23:

TATGCGGGTA CCGATCCAGA AAGTTCAGGA CGACACCAAA
ACCCTGATCA AAACCATCGT 60

TAC 63

(2) INFORMATION FOR SEQ ID NO: 24:

(i) SEQUENCE CHARACTERISTICS:
(A) LENGTH: 74 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 24:

GCGTATCAAC GACATCTCCC ACACCCAGTC CGTGAGCTCC
AAACAGAAGG TTACCGGTCT 60

GGACTTCATC CCGG 74

(2) INFORMATION FOR SEQ ID NO: 25:

(i) SEQUENCE CHARACTERISTICS:
(A) LENGTH: 58 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 25:

GTCTGCACCC GATCCTGACC CTGTCCAAAA TGGACCAGAC
CCTGGCTGTT TACCAGCA 58

(2) INFORMATION FOR SEQ ID NO: 26:

(i) SEQUENCE CHARACTERISTICS:
(A) LENGTH: 67 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 26:

ATACGCGTAA CGATGGTTTT GATCAGGGTT TTGGTGTCGT
CCTGAACTTT CTGGATCGGT 60

ACCCGCA 67

(2) INFORMATION FOR SEQ ID NO: 27:

(i) SEQUENCE CHARACTERISTICS:
(A) LENGTH: 75 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 27:

TGCAGACCCG GGATGAAGTC CAGACCGGTA ACCTTCTGTT
TGGAGCTCAC GGACTGGGTG 60

TGGGAGATGT CGTTG 75

(2) INFORMATION FOR SEQ ID NO: 28:

(i) SEQUENCE CHARACTERISTICS:
(A) LENGTH: 55 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 28:

GATCTGCTGG TAAACAGCCA GGGTCTGGTC CATTTTGGAC
AGGGTCAGGA TCGGG 55

(2) INFORMATION FOR SEQ ID NO: 29:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 29:

Val Pro Ile Gln Lys Val Gln Asp Asp Thr Lys Thr Leu Ile Lys Thr
                5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145